# EUROPÄISCHE PATENTSCHRIFT

(11) **EP 0 483 059 B1**
(45) Veröffentlichungstag und Bekanntmachung des Hinweises auf die Patenterteilung: **09.08.1995**
(21) Anmeldenummer: 91810781.4
(22) Anmeldetag: 04.10.1991
(51) Int. Cl.: A61B 1/12

(54) **Maschine zum Reinigen von schlauchfoermigen Artikeln**
Apparatus for cleaning tubular devices
Machine à laver des appareils constitués de tuyaux

(30) Priorität: 23.10.1990 CH 3386/90
(43) Veröffentlichungstag der Anmeldung: 29.04.1992
(73) Patentinhaber: HAMO AG, CH-2542 Pieterlen (CH)
(72) Erfinder: Moser, Hansruedi, CH-2532 Magglingen (CH)
(74) Vertreter: AMMANN PATENTANWAELTE AG BERN

(56) Entgegenhaltungen:
- EP-A- 0 089 605
- EP-A- 0 401 594
- DE-A- 3 323 590
- DE-A- 3 430 631
- DE-A- 3 835 861

## Beschreibung

Die Erfindung betrifft eine Maschine zum Reinigen von schlauchförmigen Artikeln, wie sie insbesondere im Laboratorium, der Industrie und auf vielen anderen Gebieten verwendet werden, z.B. in der Medizin, der Raumfahrt und der Kerntechnik. Insbesondere ist die erfindungsgemässe Maschine zum Reinigen von Endoskopen und vorzugsweise von Gastroskopen, d.h. Magenendoskopen, geeignet. Die genannte Reinigung schliesst das Desinfizieren und Trocknen der Artikel ein.

Endoskope sind Betrachtungsgeräte für das Innere von Hohlräumen des menschlichen und tierischen Körpers und gestatten oft auch die Entnahme von Gewebeproben (Biopsie) aus dem Körper; sie sind schlauch- bzw. röhrenförmige, biegsame optische Geräte, die meist durch eine natürliche Oeffnung in den Körper eingeführt werden, so dass eine lokale Inspektion der Höhlung vorgenommen werden kann. Die jeweilige Endoskopform ist den anatomischen Gegebenheiten angepasst. Am Ende eines röhrenförmigen Schaftes befindet sich eine Lichtquelle - heute ist die Lichtquelle ausserhalb des Endoskops angebracht und schickt ihr Licht durch eine Faseroptik an den Endoskopkopf - sowie ein Getriebe zur Bewegung des Endoskopkopfes in alle Richtungen am hinteren Ende . Das erzeugte Bild wird durch einen zweiten Lichtleiter nach aussen an ein Okular geleitet. Zusätzlich haben Gastroskope mehrere Kanäle zur Zufuhr und Gewinnung von Magensäften, Zufuhr von Kontrastmittel usw. Das Ganze ist in einer schlauchförmigen Hülle untergebracht.

Endoskope sind sehr teure Instrumente, so dass sie nach Gebrauch nicht weggeworfen werden. Sie müssen vielmehr gereinigt, desinfiziert und/oder sterilisiert werden.

Das Reinigen der feinen Kanäle ist schwierig. Ein einfaches Durchspülen reicht nicht aus, denn es kann vorkommen, dass der eine oder andere Kanal verstopft ist. Auch besteht die Möglichkeit, dass der äussere Mantel beschädigt ist und leckt. Schliesslich bietet auch die Trocknung der Kanäle Schwierigkeiten.

Es ist bisher eine Spülmaschine zur Reinigung von schlauchförmigen Artikeln bekannt geworden, die folgende Hauptteile aufweist:
- einen Maschinenkörper mit Spülraum, Sammelbecken für die Spülflüssigkeit, Umwälzpumpe und rotierenden Sprüharmen, alles bekannt von den üblichen Geschirrspülautomaten, sowie mit einschiebbaren Körben zur Aufnahme der Schlauchartikel;
- Anschlüsse der zu spülenden Kanäle der Schlauchartikel an Zufuhrrohre für Spülflüssigkeit; und
- Einrichtungen zur Programmierung und Steuerung des Reinigungsablaufs mit Programmwahl.

Eine derartige Spülmaschine ist aus der EP-A-0 089 605 bekannt. Sie erlaubt es darüber hinaus, die Dichtigkeit des Mantels zu prüfen. Dazu wird der Zwischenraum zwischen dem Mantel und den Kanälen, der sogenannte Mantelraum, mit einer Pressluftquelle verbunden. Ist der Mantel verletzt, so wird an der Leckstelle Blasenbildung beobachtet, wenn das Endoskop während der Prüfphase in Flüssigkeit eingetaucht ist.

Für eine verbesserte Reinigungswirkung der Spülflüssigkeit in den Kanälen ist noch vorgesehen, dem Flüssigkeitsstrom Pressluft kontinuierlich beizumischen.

Aus der EP-A-0 401 594 (nachveröffentlicht) ist ein Verfahren bekannt, wie die Dichtigkeit des Mantelraums eines Endoskops im Rahmen der Desinfizierung und Sterilisierung geprüft werden kann. Dazu wird der Ueberdruck im Mantelraum während des Desinfektionsvorgangs überwacht. Fällt der Druck unter einen vorgegebenen Minimalwert, wird der Desinfektionsvorgang wegen Undichtigkeit des Endoskops abgebrochen. Beim Durchspülen der Kanäle wird auch überwacht, ob die Spülflüssigkeit durch den Kanal hindurchtreten kann, und gegebenenfalls ein Alarm wegen verstopftem Kanal ausgelöst.

Diese Vorrichtung arbeitet halbautomatisch, d. h. es fallen eine Vielzahl manueller Tätigkeiten, wie Zugabe von Sterilisier- und Desinfektionsflüssigkeit, an.

Nachteilig an der ersten Ausführung ist u.a., dass die Dichtigkeitskontrolle in einer eigenen Betriebsphase erfolgt und per Augenschein die eventuelle Blasenbildung beobachtet werden muss. Ausserdem ist es so nicht möglich, die Dichtigkeit der Kanäle zu überprüfen. Die zweite Ausführung erlaubt zwar eine automatische Dichtigkeitsprüfung und benötigt keine eigene Dichtigkeitsprüfphase, es kann jedoch, wenn z. B. kalte Desinfektionsflüssigkeit zugesetzt wird, ein Druckabfall auftreten, der fälschlicherweise als Undichtigkeit interpretiert wird.

Auch die Durchgängigkeitsprüfung der Kanäle kann irrtümlich eine Verstopfung anzeigen, obwohl der Kanal noch eine gewisse Durchgängigkeit aufweist, und die Behinderung im Laufe der Behandlung beseitigt werden könnte.

Beide Ausführungen verlangen ausserdem, die Kanäle und den Mantelraum fest mit den entsprechenden Anschlüssen der Spülmaschine zu verbinden, was wegen der Enge des Spülraums und der darin vorhandenen Spülarme lästig ist.

Aufgabe der Erfindung war es, die bekannte Spülmaschine zu ergänzen und darüber hinaus in mehreren Punkten entscheidend zu verbessern und einen noch sichereren Betrieb als mit den bekannten Spülmaschinen zu gewährleisten, was im Sinne der Reinigung und Desinfizierung absolut vorrangig und unabdingbar ist.

Die Lösung dieser Aufgabe zerfällt in mehrere Einzelteile und Massnahmen im Zusammenhang mit den oben aufgeführten Teilen und Programmstufen; sie ist im unabhängigen Patentanspruch 1 definiert. Besondere Ausführungsformen sind in abhängige Ansprüche aufgenommen.

In der nun folgenden Beschreibung eines Ausführungsbeispiels der erfindungsgemässen Maschine in Form einer Endoskop-Spülmaschine werden gleichzeitig die Nachteile der bisherigen Konstruktion und ihre Ueberwindung besprochen.

In der Zeichnung ist eine nach der erfindungsgemässen Lehre gebaute Spülmaschine dargestellt. Es zeigen:
Fig. 1 ein Schema der Spülmaschine und
Fig. 2 einen Koppler für Anschlussleitungen im Längsschnitt.

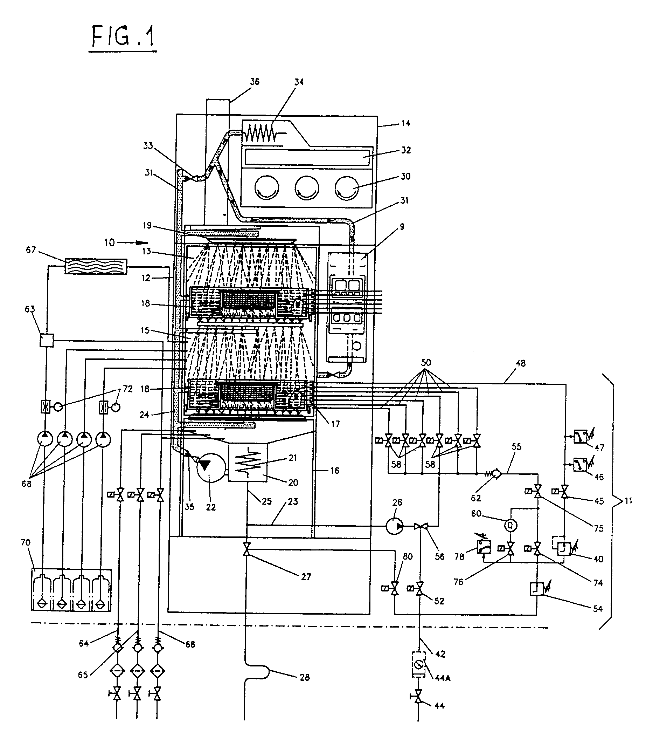

Die Spülmaschine (Fig. 1) besitzt in bekannter Weise einen Spülmaschinenkörper 10, der in drei vertikal übereinanderliegende Hauptabschnitte eingeteilt ist: Ein Mittelteil 12, das den eigentlichen Reinigungsraum darstellt, ein Oberteil 14 und ein Unterteil 16.

Das Mittelteil 12 besteht aus einem oder mehreren oder, wie gezeigt, zwei im wesentlichen identischen Abschnitten 13 und 15, in welchen sich Körbe 18 befinden, die die zu reinigenden Endoskope enthalten und von vorn in das Mittelteil 12 eingeschoben sind. In an sich bekannter Weise sind rotierende Sprüharme 19 vorhanden. Seitlich am Mittelteil 12 ist noch die elektronische Steuerung 9 angebracht. Diese Steuerung 9 mit einem Bedienungs- und Anzeigefeld gestattet die manuelle Auswahl von Programmen und zeigt den Betriebszustand der Maschine sowie auch eventuelle Störungen an. Die Steuereinheit 9 enthält alle zur Steuerung und Ueberwachung des Betriebsablaufs erforderlichen Einrichtungen, darunter auch einen Mikroprozessor.

Die von den Sprüharmen 19 verspritzte Reinigungsflüssigkeit gelangt ins Unterteil 16, wo sie sich im Sammelbecken oder Sumpf 20 sammelt, der noch mit einem Heizkörper 21 versehen ist; er dient zur Nachheizung der Flüssigkeit. Diese wird von der Umwälzpumpe 22 durch das Rohr 24 unter Druck wieder den Sprüharmen 19 und anderen, feststehenden Sprühdüsen zugeführt.

Im Gegensatz zur bekannten Lösung, bei der die Leitung 24 eine einfache Abzweigung aufweist und für die noch zu beschreibende Innenreinigung der Endoskopkanäle weitere Druckpumpen, nämlich für jeden Kanal eine, besitzt, zweigt gemäss Erfindung vom Sumpf 20 bzw. der Ablaufleitung 25 eine Leitung 23 zur gemeinsamen Spülpumpe 26 ab.

Im Maschinenunterteil 16 sind weiterhin Ventile, Regler und andere Vorrichtungen angebracht, die allgemein mit 11 bezeichnet sind und weiter unter beschrieben werden. Ausserdem ist noch ein magnetgesteuertes Ablassventil 27 in die Ablaufleitung 25 eingebaut, durch das der Ablauf der Reinigungsflüssigkeit zum Siphon 28 gesteuert wird.

Im Oberteil 14 befinden sich Ventilatoren 30 für Trockenluft, die durch ein Feinfilter 32 und an einem Luftheizkörper 34 vorbei in die Rohrleitungen 31 gedrückt wird, wenn das Luftventil 33 offen und das Ventil 35 (im Unterteil 16) der Umwälzpumpe 22 geschlossen ist und die Trockenphase durchzuführen ist.

Die Körbe 18, welche jeweils ein Endoskop aufnehmen, sind bevorzugt als geschlossene Kästen mit einer beweglichen, nicht dargestellten Seitenklappe ausgeführt. Sie enthalten im Inneren Rohrstutzen, auf die je ein Anschlussschlauch des zu reinigenden Kanals aufgesteckt wird. Diese Stutzen sind ebenfalls nicht dargestellt. Es wird weiter unten beschrieben, wie die Verbindung mit den Spülleitungen hergestellt wird.

Die Kästen 18 sollen die Gesamtreinigung des Aeusseren der Endoskope gestatten, nämlich durch vollständiges Fluten ("Einlegen"). Es muss aber auch ein Ablauf vorhanden sein, damit sich jeder Kasten 18 am Ende jedes Spülvorganges rasch nach unten entleert.

Es sollen nun die Zusatzeinrichtungen 11 näher besprochen werden.

Die erste Gruppe der Zusatzeinrichtungen betrifft die periodische Druckprüfung des Aussenmantels des Endoskops. Es hat sich erwiesen, dass der Mantel des Endoskops, das nicht dargestellt ist, von innen höchstens auf einen Druck von 0,2 bar gebracht werden darf.

Kernstück der Druckprüfungseinrichtung zur Dichtigkeitskontrolle des Endoskopmantels ist ein automatisches Druckminderventil 40. Dieses ist über ein Druckminderventil 54 für die Kanalprüfung mit einer Druckluftleitung 42 verbunden, die über eine Wartungseinheit 44A und ein Absperrventil 44 an eine nicht gezeichnete Druckluftquelle, beispielsweise von 2,5 bar, angeschlossen ist. Das Druckminderventil 40 hält in der Anschlussleitung 48 den Nominaldruck aufrecht, z.B. 0,2 bar, Ueberdruck wird abgelassen. Dem Druckminderautomaten 40 ist ein Magnetventil 45 nachgeschaltet. Dieses Ventil und auch die übrigen aktiven Bestandteile der Zusatzeinrichtungen 11 sind mit der Steuer-, Kontroll- und Programmiereinheit 9 durch nicht dargestellte Leitungen verbunden; diese Verbindungen werden im Folgenden nicht jedesmal erwähnt.

In die Leitung 48, die zum Anschluss des Mantelraumes des Endoskops führt, sind weiterhin zwei Druckschalter 46 und 47 eingebaut. Dabei spricht der eine Druckschalter, z.B. 47, auf einen unter dem Prüfdruck liegenden Druck und der andere Druckschalter, demnach 46, auf einen darüberliegenden Druck an.

Diese Anordnung aus den Teilen 40,45,46,47,48 arbeitet im Zusammenwirken mit der Steuereinheit 9 folgendermassen, wobei Zeiten und Drücke nur als Beispiele dienen: Der Nominaldruck (Prüfdruck) betrage 0,18 bar, der obere Schaltdruck 0,20 bar und der untere Schaltdruck 0,16 bar. Zunächst öffnet das Magnetventil 45 während 3 Sekunden. In der Leitung 48 und im Mantel des Endoskops, der einen geschlossenen Raum darstellt, baut sich der Prüfdruck von 0,18 bar auf, geregelt vom Regler 40. Sodann schliesst das Magnetventil 45 wieder.

Bei dichtem Mantel und Erwärmung des Endoskops in der Spülmaschine steigt der Druck im Mantel. Sollte er 0,20 bar erreichen, so öffnet, veranlasst vom Schalter 46, das Ventil 45, und der Regler 40 sorgt für den Abbau des Druckes auf 0,18 bar. Bei dichtem Mantel und Abkühlung des Endoskops in der Spülmaschine (Einlauf von Kaltwasser oder kaltem Reinstwasser) sinkt der Luftdruck im Endoskopmantel. Solange das Einfüllventil für Kaltwasser oder Reinstwasser geöffnet ist (Leitungen 64 bzw. 66) und dabei der Prüfluftdruck unter 0,16 bar sinkt, öffnet, veranlasst vom Schalter 47, das Ventil 45 während 2 Sekunden, und der Regler 40 sorgt für den Druckanstieg auf 0,18 bar.

Ist der Endoskopmantel oder einer der Kanäle des Gerätes undicht, so fällt der Druck, und bei einem Druckabfall auf unter 0,16 bar wird vom Schalter 47 Alarm ausgelöst und die Spülmaschine stillgesetzt. Diese Phase der Druckprüfung dauert z.B. 3 Minuten, wonach der geschilderte Zyklus während der ganzen Spüldauer wiederholt wird. So wird sichergestellt, dass kein Wasser ins Endoskop gelangt und dieses gegebenenfalls repariert werden kann.

Die angegebenen Druck- und Grenzwerte sowie die Zeiten können selbstverständlich frei gewählt und den Anforderungen angepasst werden.

Die in Fig. 1 weiter gezeigten zusätzlichen Leitungen 50 - normalerweise drei bis sieben - sind mit entsprechenden Kanälen des Endoskops über ein automatisches Anschlussstück 17 verbunden, das weiter unten beschrieben wird. Im Gegensatz zum Mantel sind diese Kanäle offen; hier wird keine Dichtigkeitsprüfung, sondern eine Durchgangsprüfung gefordert und durchgeführt, und zwar mit Luft vor und nach dem Waschen und Spülen.

Zu diesem Zweck ist in der Reinstluftleitung 42 ein Druckverminderer 54 vorgesehen. Das Ventil 52 sorgt dafür, dass ein Absperrventil 56 hinter der Kanal-Spülpumpe 26 in der Spülleitung 23 schliesst und daher die folgenden Apparateteile nicht mit Spülflüssigkeit versorgt werden.

Die Spülleitung 23 verzweigt sich hinter dem Absperrventil 56 zu mehreren Ventilen 58, welche die Leitungen 50 zu den Endoskopkanälen öffnen oder verschliessen. Es sind dies nach weiterer Erfindung Schlauchquetschventile, die nicht verschmutzen und sich selbst reinigen und von kräftigen Magneten angetrieben werden.

Für die Durchgangsprüfung der Endoskopkanäle wird nun das Ventil 74 geschlossen und das Ventil 76 geöffnet. Wenn der angeschlossene Endoskopkanal nicht verstopft ist, so strömt Luft über einen in die Prüfleitung eingebauten Schwebekörper-Magnetschalter 60, die Ventile 75 und 62 und das betreffende Kanalventil 58 durch den Endoskopkanal. Anschliessend wird der geprüfte Kanal durch Schliessen des Ventils 58 gesperrt und ein anderer Kanal geprüft. Sollte ein Kanal verstopft sein, so wird der Magnetschalter 60 nicht freigegeben, der verstopfte Kanal wird an einem Anzeigefeld der Steuereinheit 9 angezeigt und die Maschine stillgesetzt.

Nach der Durchgangsprüfung kann der Reinigungsvorgang beginnen, der sich grundsätzlich von den bekannten Methoden nicht unterscheidet. Wie üblich sind mehrere Programme vorgesehen und wählbar.

Zunächst werden durch die Leitungen 64, 65 und 66 je nach Programm Kaltwasser, Warmwasser oder Reinstwasser (letzteres nur zum abschliessenden Spülen) in das Maschinenunterteil eingespeist und im Sumpf 20 von der Heizung 21 nach Bedarf erwärmt. Die Umwälzpumpe 22 drückt die Reinigungsflotte in die Sprüharme 19 und in andere, feste Sprühvorrichtungen. Der Zusatz von Reinigungsmittel, Neutralisationsmittel, sog. Instrumentenmilch, und Desinfektionsmittel geschieht mittels Pumpen 68, die die entsprechenden Flüssigkeiten aus einer Behälterbatterie 70 in das Mittelteil 12 der Spülmaschine pumpen, wo sie sich mit dem umlaufenden Spülwasser vermischen. Mit Hilfe von Durchflussüberwachungsgeräten 72 wird der Durchfluss einiger oder aller Zusätze überwacht und, wenn nötig, gemessen. In der Regel genügt es jedoch, die nötigen Mengen an Zusätzen durch Zeitgabe der Pumpen 68, die als Dosierpumpen ausgestaltet sind, zu dosieren.

Das Desinfektionsmittel, das aus der ganz links in Fig. 1 gezeigten Flasche der Batterie 70 zugepumpt wird, gelangt nicht direkt in die Spülmaschine, sondern als gebrauchsfertige, sterile, verdünnte Lösung. Zu diesem Zweck ist ein Mischpunkt 63 vorgesehen, dem einerseits Desinfektionskonzentrat und andererseits Reinstwasser aus der Leitung 66 zuströmt. Nach Vermischen der beiden Flüssigkeiten, wobei das Mischungsverhältnis einstell-und regelbar gemacht werden kann, gelangt die verdünnte Mischung in ein Sterilfilter 67; eine derartige Anordnung bildet den Gegenstand eines nicht veröffentlichten Vorschlags.

Als Sterilfilter 67 wird vorteilhafterweise ein Membranfilter eingesetzt, der einen hohen Wasserfluss zulässt. Besonders vorteilhaft werden High-Flux-Industriefilter eingesetzt, wie sie bei der üblichen Hämodialyse für die Sterilfiltration zum Einsatz kommen. Derartige Filter sind aufgrund ihrer Materialeigenschaften hydrophil und bestehen beispielsweise aus Polysulfon, das mit Hilfe von Polyvinylpyrrolidon hydrophilisiert wurde, Celluloseacetat, Polyacrylnitril und dgl. Sie haben üblicherweise eine Membranoberfläche zwischen 1 und 3 m² und liegen gewöhnlich in Form eines Hohlfaserfilters vor, der ca. 9'000-10'000 Hohlfasern in einem im wesentlichen zylindrischen Gehäuse enthält. Derartige Hohlfasermembranen haben einen Innendurchmesser von etwa 0,2 mm, eine Wandstärke von ca. 20-30 »m und eine mittlere Porengrösse unterhalb 0,5 »m, insbesondere unterhalb 0,1 »m.

Die erfindungsgemässe Vorrichtung weist nunmehr den Vorteil auf, dass das vor der Spülkammer 15 angeordnete Sterilfilter 67 ein Einschleppen von Keimen in die Spülkammer sicher verhindert, und es bleiben sämtliche mit flüssigem Desinfektionsmittel in der Spülmaschine behandelten Geräte auch nach dem Spülen mit sterilem Frischwasser, wie es ausgangs des Sterilfilters erhalten wird, sicher steril.

Bei den Reinigungsprogrammen werden die Endoskope von aussen nicht nur abgespritzt, sondern, da sie sich in Kästen 18 und nicht in Körben befinden, vollständig in Reinigungsflüssigkeit eingelegt. Dies ist ein wichtiger Vorteil der Erfindung, da es sich gezeigt hat, dass ein blosses Abspritzen nicht genügt.

Gleichzeitig werden die Endoskopkanäle gereinigt, indem alle Kanalventile 58 geöffnet sind und die Pumpe 26 bei geöffnetem Ventil 56 die Reinigungsflotte über die Leitung 23 zuführt. Periodisch wird das Ventil 52 geöffnet, wodurch das Ventil 56 gegen die Pumpe 26 schliesst und ein Druckluftstoss über die Leitung 55, die Ventile 58 und die Leitungen 50 in die Endoskopkanäle gegeben wird. Diese periodischen Luftdruckstösse unterstützen die Reinigung der Kanäle entscheidend.

Nach jedem Spülvorgang, d.h. im allgemeinen Vorreinigung, Reinigung, Desinfektion, Schlussspülung und eventuell Trocknen, werden die Endoskopkanäle durch Ausblasen entleert. Dazu werden bei geschlossenem Ventil 56 die Luftventile 74 und 75 geöffnet. Der angeschlossene Druckminderer 54 setzt den Luftdruck auf einen geeigneten Wert herab; das Ventil 75 wird von einem Druckschalter 78 geschlossen, wenn sich in der Leitung 55 ein unzulässig hoher Druck aufbauen sollte. Beim Ausblasen sind normalerweise alle Kanalventile 58 offen.

Am Schluss jeder Reinigungsphase kann die Spülflotte über das pneumatisch betätigte Ventil 27 (Steuerung vom Luftventil 80) angelassen werden.

Die äussere Trocknung der Endoskope wird mit gefilterter Warmluft bewirkt, die von den Ventilatoren 30 über das Filter 32 und die Heizung 34 in die Maschine eingeblasen wird (Leitungen 31). Die Abluft entweicht durch das Rohr 36.

Es sei wiederholt, dass während der ganzen Reinigungsprozedur die Dichtigkeit des Endoskopmantels geprüft wird, wie es oben ausführlich beschreiben ist.

An den Kästen 18 für die Endoskope ist, wie erwähnt, je eine seitliche Klappe angebracht, die sich nach innen öffnet. Bei eingelegtem Endoskop kann die Klappe nicht geöffnet werden. Sie kann an der Oberseite mechanische, optische oder magnetische Markierungen besitzen, welche beim Einschieben des Kastens mit entsprechenden Fühlern an der Maschine zusammenwirken. Dies gestattet eine automatische Programmwahl. Bei herausgenommenem Endoskop kann die erwähnte Klappe durch Schwenken um eine senkrechte Achse geöffnet werden, die Magnetmarkierungen kommen ausser Wirkung, und infolge des nun offenen Kastens kann dieser gründlich thermisch desinfiziert werden.

Der Kasten mit den erwähnten Einrichtungen ist in der Zeichnung nicht dargestelt, da die detaillierte Ausbildung des geschilderten Aufbaus vom Fachmann verstanden wird.

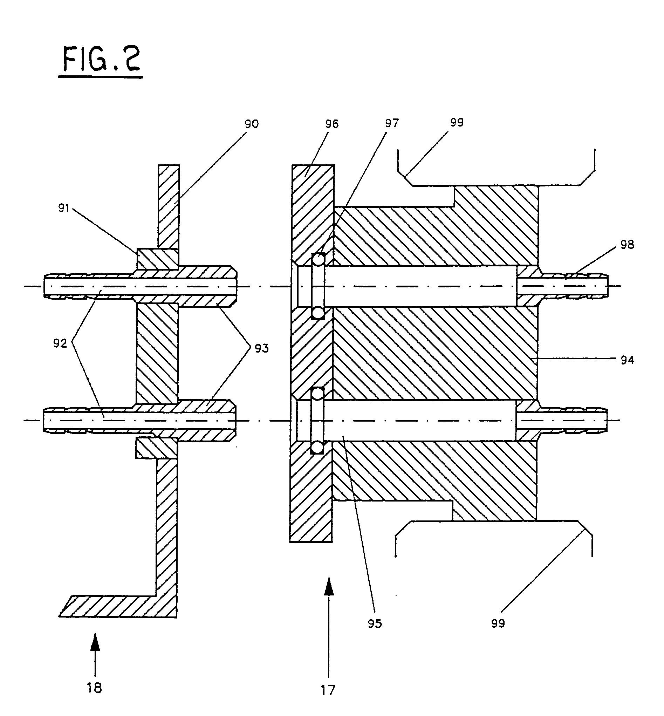

In der Zeichnung zeigt Fig. 2 schematisch einen Längsschnitt eines Teiles des Korbes 18 und des Anschlussstückes (oder Kopplers) 17.

In den hinteren Bereich einer Seitenwandung 90 des Korbes 18 (oder in der Hinterwand des Korbes) ist ein Verbindungsstück 91, z.B. mit kreisrundem Querschnitt, dicht eingesetzt. Dieses Verbindungsstück 91 ist durchbohrt, und die Bohrungen enden auf der Innenseite des Korbes 18 in Schlauchstutzen 92, an die die Kanäle des zu reinigenden Endoskops (nicht dargestellt) angeschlossen werden. Auf der Aussenseite sind gerade, glatte, vorzugsweise vorn abgerundete Stutzen 93 vorgesehen. Die Anzahl der Bohrungen mit den Stutzen 92 und 93 ist an die Zahl der anzuschliessenden Kanäle des Endoskops, einschliesslich dem Mantel, angepasst; sie beträgt mindestens eins und in der Regel sieben, wie in Fig. 1 dargestellt ist (Leitungen 48 und 50). In Fig. 2 sind der Einfachheit halber nur zwei Anschlüsse dargestellt.

Zum Verbindungsstück 91 passend ist im Mittelteil 12 der Spülmaschine 10 ein Koppler 17 angeordnet. Dieser Koppler 17 ist derart plaziert, dass bei korrekt in die Maschine eingeschobenem Korb 18 durch seitliche Verschiebung des Kopplers 17 eine direkte, zuverlässige, spannungsfreie Verbindung mit dem Verbindungsstück 91 hergestellt werden kann, und zwar automatisch, wenn nicht gezeichnete Sensoren die korrekte Endstellung des Einschubs des Korbes 18 signalisieren.

Der Koppler 17 besteht aus einem zylindrichen Block 94, der so viele Bohrungen 95 aufweist, wie Stutzen 93 am Korb 18 vorgesehen sind. Die Bohrungen 95 fluchten in Endstellung des Korbes 18 mit den Stutzen 93. Die Bohrungen 95 enden in einer Stirnscheibe 96 des Blockes 94, wo geeignete Dichtungsmittel für die zugehörigen Stutzen 93 angebracht sind, beispielsweise O-Ring-Dichtungen 97 aus einem selbstschmierenden Material. An der Rückseite des Blockes 94 sind wiederum Schlauchstutzen 98 als Verlängerung der Bohrungen 95 angebracht; sie dienen zur Verbindung mit den Leitungen 48 und 50 (Fig. 1). Der Umfang des Blockes 94 ist als doppeltwirkender Pneumatikkolben ausgeführt und wirkt mit einem sehr schematisch dargestellten Pneumatikzylinder 99 zusammen. Zum Verständnis der Konstruktion nicht unmittelbar erforderliche Teile wie Führungen und Verdrehungssicherung des Blockes 94, Anschläge zur Hubbegrenzung, Leitungen, Sensoren usw. sind nicht dargestellt. Die Stirnscheibe 96 kann auch mit dem Block 94 ein Stück bilden.

Der Koppler arbeitet wie folgt. Nach korrektem Einschieben des Korbes 18 in die Maschine 10 fluchten die Stutzen 93 mit den Bohrungen 95. Nach Erregen entsprechender Fühler (nicht dargestellt) verschiebt der Pneumatikzylinder 99 den Koppler 17 nach links in Fig. 2 (und 1), bis die Stirnscheibe 96 an die Korbwandung 90 zum Anliegen kommt. Dabei treten die Stutzen 93 in die Bohrungen 95 ein und werden durch die O-Ringe 97 abgedichtet.

Damit ist die Verbindung der Leitungen 48 und 50 mit den zu reinigenden Kanälen des Endoskops hergestellt.

Nach beendeter Spülung und Trocknung der Kanäle des Endoskops wird der Koppler 17 automatisch zurückgezogen, und zwar im wesentlichen in die in Fig. 2 gezeigte Stellung, und der Korb 18 kann aus der Maschine 10 ausgefahren werden.

Es wurde im Vorstehenden bereits angedeutet, dass für einzelne Teile oder Vorgänge andere als die beschriebenen möglich sind. Die Erfindung ist nicht auf das im Beispiel konkret Beschriebene beschränkt, sondern kann im Rahmen des Beanspruchten nach Wissen und Können des Fachmannes abgewandelt werden, ohne ihren Schutz einzubüssen. Beispielsweise können die Stutzen am Verbindungsstück gemäss Fig. 2 und die Bohrungen im Kopplerblock gegeneinander ausgetauscht werden. Die Anschlusselemente am Kopplerblock können Schraubanschlüsse sein, usw.

## Patentansprüche

1. Maschine zum Reinigen von schlauchförmigen Artikeln, insbesondere Endoskopen, wobei der Artikel einen schlauchförmigen, dichten Mantel und zumindest einen innerhalb des Mantels verlaufenden Kanal mit einer eigenen, dichten Hülle aufweist, wodurch ein Mantelraum zwischen Mantel und Kanälen gebildet wird, mit
- einem Maschinenkörper (10) mit Spülraum (12), Sammelbecken (20) für die Spülflüssigkeit, Umwälzpumpe (22) und rotierenden Sprüharmen (19), und mit mindestens einem einschiebbaren Korb (18) zur Aufnahme des Spülguts;
- Anschlüssen (92, 93) der Kanäle der zu spülenden schlauchförmigen Artikel an Zufuhrrohre (50) für Spülflüssigkeit;
- mit den Zufuhrrohren (50) verbundene erste (23, 26) und zweite (42, 54, 74, 76, 75, 55, 62) Vorrichtungen, um Spülflüssigkeit bzw. Druckluft in die Zufuhrrohre einspeisen zu können, wobei diese zwei Vorrichtungen über getrennte Stellglieder (56, 75) zur Steuerung der Einspeisung verfügen;
- mindestens einem Anschluss an den Mantelraum der zu spülenden schlauchförmigen Artikel;
- eine mit dem Anschluss des Mantelraums verbundene erste (48, 45, 46, 47, 54) Einrichtung zur Erzeugung eines Luftüberdrucks im Mantel gegenüber den Kanälen und der Umgebung des Artikels, wobei Drucküberwachungsmittel (46, 40) vorhanden sind, um den Überdruck auf einen für den Artikel ungefährlichen Druckhöchstwert zu begrenzen;
- zweite Einrichtungen zur Programmierung und Steuerung des Reinigungsablaufs mit Programmwahl;
**gekennzeichnet durch**
(A) in der ersten Einrichtung vorhandene Mittel (47, 45) zur Dichtigkeitsprüfung des Mantelraumes, wobei die Mittel einen Manteldruckschalter (47) für den Druck im Mantelraum umfassen, der bei der Durchführung der Dichtigkeitsprüfung auf Unterschreiten eines ersten Mindestdruckes im Mantelraum anspricht, woraus auf eine Undichtigkeit des Mantels und/oder einer Kanalhülle schliessbar ist, und diese Dichtigkeitsprüfung in Intervallen während der gesamten Laufdauer des Reinigungsablaufs wiederholbar ist;
(B) Mittel (76, 60) zur pneumatischen Durchgangsprüfung der Kanäle des zu reinigenden Artikels;
(C) Mittel (52, 56, 55, 62) zur Erzeugung von pneumatischen Druckstössen auf die Spülflüssigkeit in den Zuführrohren zur Unterstützung der Reinigungswirkung; und
(D) Mittel (17, 18) zur automatischen Verbindung der zu reinigenden Artikel mit den Mitteln (A), (B), und (C) vor Beginn des Spülvorgangs.

2. Maschine nach Anspruch 1, dadurch gekennzeichnet, dass die Mittel (A) folgende Bestandteile aufweisen:
- ein automatisches pneumatisches Druckminderventil (4) mit einer Einrichtung zur Konstanthaltung des Gasdrucks auf der Niederdruckseite;
- ein Magnetventil (45) zur periodisch gesteuerten Betätigung;
- einen Druckschalter (46), der auf einen Ueberdruck über den genannten Gasdruck anspricht;
- einen Druckschalter (47), der auf einen Unterdruck unter dem genannten Gasdruck anspricht; und
- eine Leitung (48) zur Verbindung mit dem Mantelraum des zu reinigenden Artikels.

3. Maschine nach Anspruch 1 oder 2, dadurch gekennzeichnet, dass die Mittel (B) folgende Bestandteile aufweisen:
- einen Gasdruckminderer (54) zur Lieferung eines Prüf-Gasdruckes;
- ein Prüfventil (76) zur steuerbaren Oeffnung und Schliessung zwecks Durchführung der Durchgangsprüfung;
- ein pneumatisch betätigtes Absperrventil (56) zum Absperren von Spülflüssigkeit zu den zu prüfenden Kanälen des genannten Artikels;
- steuerbare Ventile (58) in Leitungen (50) zu den Kanälen des zu reinigenden Artikels; und
- einen Durchflussfühler (60).

4. Maschine nach Anspruch 3, dadurch gekennzeichnet, dass die genannten steuerbaren Ventile (58) magnetisch betätigte, selbstreinigende Schlauchquetschventile sind.

5. Maschine nach Anspruch 3 oder 4, dadurch gekennzeichnet, dass der Durchflussfühler (60) ein Schwebekörper-Magnetschalter ist, der einen fehlenden oder unzureichenden Durchgang als Störung signalisiert.

6. Maschine nach einem der vorstehenden Ansprüche, weiterhin gekennzeichnet durch Mittel zur Durchflusskontrolle mindestens einer zuzuführenden Reinigungsflüssigkeit.

7. Maschine nach Anspruch 6, dadurch gekennzeichnet, dass die genannten Mittel Durchfluss-Kontrollgeräte (72) im Zusammenwirken mit Pumpen (68) sind, die in die gleiche Leitung eingebaut sind.

8. Maschine nach einem der vorstehenden Ansprüche, dadurch gekennzeichnet, dass die Mittel (C) ein pneumatisch betätigtes Absperrventil (56) umfassen, das über ein steuerbares Ventil (52) periodisch geöffnet und geschlossen wird und in die Spülmittelleitung (23) eingebaut ist, sowie eine Druckluftzuleitung (55), durch die bei geschossenem Absperrventil (56) Druckluft in die Spülmittelleitung einspeisbar ist.

9. Maschine nach einem der vorstehenden Ansprüche, dadurch gekennzeichnet, dass die Mittel (D) folgende Bestandteile aufweisen:
- ein Verbindungsstück (91) in einer Wandung (90) des genannten Korbes (18), das an der Korbinnenseite mit mindestens einem Schlauchstutzen (92) zum Anschluss des zu reinigenden Artikels und an der Korbaussenseite mit mindestens einem geraden Stutzen (93) sowie mindestens einer Durchgangsbohrung versehen ist;
- einen maschinenseitig angeordneten Kopplerblock (17) mit mindestens einer mit dem genannten geraden Stutzen (93) fluchtenden Bohrung (95), die korbseitig mit Dichtungsmitteln (97) und maschinenseitig mit Anschlusselementen (98) ausgerüstet ist; und
- einen doppeltwirkenden Pneumatikzylinder (99) zum horizontalen Bewegen des Kopplerblocks (17) über die geraden Stutzen (93) des Verbindungsstückes (91), und in Gegenrichtung zu deren Freigabe.

10. Maschine nach Anspruch 1 und 6, dadurch gekennzeichnet, dass in die Leitung für die Zufuhr von Desinfektionsmittel als Reinigungsflüssigkeit ein Mischpunkt (63) eingebaut ist, wo sich Desinfektionsmittel und Reinstwasser vermischen, und dass eine das Gemisch führende Leitung über ein Sterilfilter (67) zum Innenraum (15) der Maschine führt.

11. Maschine nach einem der vorstehenden Ansprüche, dadurch gekennzeichnet, dass der Korb (18) als Kasten ausgeführt ist, der soweit wasserrückhaltend ist, dass er beim Spülvorgang geflutet wird.

12. Maschine nach Anspruch 11, dadurch gekennzeichnet, dass der Kasten (18) Markierungen und die Maschine (10) damit zusammenwirkende Fühler zur automatischen Programmwahl aufweist.

13. Maschine nach Ansprüchen 11 und 12, dadurch gekennzeichnet, dass der Kasten (18) eine seitliche Klappe aufweist, die in Offenstellung mindestens eine Markierung in Ausserwirkungsstellung mit dem Fühler bringt.

14. Maschine nach Anspruch 12, dadurch gekennzeichnet, dass die genannten Markierungen Magnetmarkierungen sind.

15. Verwendung der Maschine nach einem der vorstehenden Ansprüche zur Prüfung und Reinigung von Endoskopen, insbesondere von Gastroendoskopen.

## Claims

1. Apparatus for cleaning tubular articles, in particular endoscopes, said articles containing a tubular, impervious envelope and at least one channel within the envelope having an own, impervious sheathing, an envelope space being formed between the envelope and the channels, said apparatus comprising
- a machine body (10) containing a washing space (12), collecting basins (20) for the washing liquid, a circulating pump (22) and rotating washing arms (19), and further containing at least one insertable cage (18) for receiving the articles to be washed;
- connections (92, 93) for connecting the channels of the tubular articles to be washed to feeding tubes (50) for washing liquid;
- first (23, 26) and second (42, 54, 74, 76, 75, 55, 62) appliances, connected to the feeding tubes (50), for feeding washing liquid or compressed air, respectively, into the feeding tubes, these two appliances having separate control elements (56, 75) for controlling the feed supply;
- at least one connection to the envelope space of the tubular articles to be washed;
- a first device (48, 45, 46, 47, 54) connected to the envelope space connection for producing an air over-pressure within the envelope with respect to the channels and the environment of the article, pressure monitoring devices (46, 40) being provided for limiting the overpressure to a maximum value which is harmless for the article;
- second devices for programming and controlling the washing sequences with programme selection;
characterised by
(A) means (47, 45) in the first device for a leak test of the envelope space, these means comprising an envelope pressure switch (47) for the pressure within the envelope space, said switch responding to a falling down of the pressure below a first minimum pressure within the envelope space during the leak test, this fact indicating a leak of the envelope and/or of a channel sheathing, said leak test being repeatable during the whole duration of the washing procedure;
(B) means (76, 60) for a pneumatic free passage test of the channels of the article to be cleaned;
(C) means (52, 56, 55, 62) for the production of pneumatic pressure shocks on the washing liquid within the feeding tubes, in order to improve the cleaning effect; and
(D) means (17, 18) for the automatic connection of the articles to be cleaned to the means (A), (B) and (C) before the start of the washing procedure.

2. Apparatus according to claim 1, characterised in that the means (A) comprise the following elements:
- an automatic pneumatic pressure reducing valve (4) having a device for keeping constant the gas pressure on the low pressure side;
- a magnet valve (45) for a periodically controlled actuation;
- a pressure switch (46) responsive to an overpressure of said gas pressure;
- a pressure switch (47) responsive to an underpressure below said gas pressure; and
- a conduit (48) for the connection with the envelope space of the article to be cleaned.

3. Apparatus according to claim 1 or 2, characterised in that the means (B) comprise the following elements:
- a gas pressure reducer (54) for supplying a test gas pressure;
- a test valve (76) for the controllable opening and closing in order to run the free passage test;
- a pneumatically operated cut-off valve (56) for cutting off the washing liquid from the channels to be tested of the said apparatus;
- controllable valves (58) in conduits (50) leading to the channels of the article to be cleaned; and
- a free passage sensor (60).

4. Apparatus according to claim 3, characterised in that the said controllable valves (58) are magnetically operated and self-cleaning hose squeezing valves.

5. Apparatus according to claim 3 or 4, characterised in that the free passage sensor (60) is a rotameter magnet switch that indicates an obstructed or insufficient passage as a failure.

6. Apparatus according to any one of the preceding claims, further characterised by means for the control of the flow of at least one washing liquid to be supplied.

7. Apparatus according to claim 6, characterised in that the said means are flow control devices (72) in co-operation with pumps (68) which are mounted in the same conduit.

8. Apparatus according to any one of the preceding claims, characterised in that the means (C) comprise a pneumatically operated cut-off valve (56) which is periodically opened and closed by a controllable valve (52) and is mounted into the rinsing liquid conduit (23), and a compressed air supply tube (55) through which compressed air can be fed into the rinsing conduit when said cut-off valve (56) is closed.

9. Apparatus according to any one of the preceding claims, characterised in that the means (D) comprise the following elements:
- a connecting part (91), mounted in a wall (90) of the said cage (18), which is provided at the inner cage side with at least one hose fitting (92) for the connection of the article to be cleaned, and at the outer cage side with at least one straight connection piece (93) and at least one through bore;
- a coupling block (17) disposed in the apparatus having at least one bore (95) aligned to said straight connection piece (93) which bore is equipped with sealing means (97) at the cage side and with connecting elements (98) at the apparatus side; and
- a double action pneumatic cylinder (99) for a horizontal displacement of the coupling block over the straight connection pieces (93) of the connecting part (91), and in inverse direction for disengaging the latter.

10. Apparatus according to claim 1 and 6, characterised in that a mixing point (63) is mounted in the conduit for the supply of disinfecting agent as a cleaning liquid, disinfecting agent and high purity water being mixed at this mixing point, and that a conduit transporting this mixture leads through a sterilising filter (67) to the interior space (15) of the apparatus.

11. Apparatus according to any one of the preceding claims, characterised in that the cage (18) is made in the form of a box which has a water retention ability such that it is flooded during the washing step.

12. Apparatus according to claim 11, characterised in that the box (18) comprises marks, and the apparatus is provided with sensors co-operating with said marks for an automatic programme selection.

13. Apparatus according to claims 11 and 12, characterised in that the box (18) has a lateral flap which, when open, brings at least one mark out of coaction with the sensor.

14. Apparatus according to claim 12, characterised in that the said marks are magnetic marks.

15. Use of the apparatus according to any one of the preceding claims for the testing and cleaning of endoscopes, particularly gastro-endoscopes.

## Revendications

1. Machine pour laver des articles tubulaires, notamment endoscopes, le dit article comportant une enveloppe étanche tubulaire et au moins un canal à l'intérieur de l'enveloppe ayant lui-même une propre gaine étanche, un espace vide étant formé entre l'enveloppe et les canaux, la machine comprenant
- un corps de machine (10) renfermant un espace de lavage (12), un bassin collecteur (20) pour le liquide de lavage, une pompe de circulation (22) et des bras gicleurs (19), et au moins une cage rétractable (18) pour recevoir l'article à laver;
- des connecteurs (92, 93) pour relier les canaux des articles tubulaires à laver à des tubes d'amenée (50) du liquide de lavage;
- des premiers (23, 26) et seconde (42, 54, 74, 76, 75, 55, 62) dispositifs, reliés aux tubes d'amenée (50), pour alimenter les tubes d'amenée en liquide à laver ou en air comprimé, respectivement, ces deux dispositifs étant équipés d'organes de commande séparés (56, 75) pour commander cette alimentation;
- au moins une connexion au vide de l'enveloppe des articles tubulaires à laver;
- une première installation (48, 45, 46, 47, 54) reliée à la connexion du vide de l'enveloppe pour produire une surpression d'air dans l'enveloppe par rapport aux canaux et à l'environnement de l'article, des moyens de surveillance de pression (46, 40) étant prévus pour limiter la surpression à une valeur maximum sans danger pour l'article;
- des secondes installations pour programmer et commander le déroulement de lavage comprenant une sélection de programmes;
caractérisée par
(A) des moyens prévus dans la première installation (47, 45) pour contrôler l'étanchéité du vide de l'enveloppe, ces moyens comprenant un interrupteur pour la pression dans le vide de l'enveloppe (47) qui réagit lors du contrôle de l'étanchéité sur une baisse d'une première pression minimum dans le vide de l'enveloppe permettant de conclure à une fuite dans l'enveloppe et/ou d'une gaine de canal, ce contrôle d'étanchéité pouvant être répété par intervalles pendant la durée entière de la procédure de lavage;
(B) des moyens (76, 60) pour le contrôle par voie pneumatique du passage libre dans les canaux de l'article à nettoyer;
(C) des moyens (52, 56, 55, 62) pour produire des chocs pneumatiques sur le liquide de lavage dans les tubes d'amenée afin de supporter l'effet nettoyant; et
(D) des moyens (17, 18) pour le raccord automatique des articles à nettoyer avec les moyens (A), (B) et (C) avant la procédure de lavage.

2. Machine selon la revendication 1, caractérisée en ce que les moyens (A) comprennent les éléments suivants:
- une soupape réductrice pneumatique automatique (4) ayant un dispositif pour tenir constante la pression du gaz côté basse pression;
- une vanne magnétique (45) étant actionnée par commande périodique;
- un interrupteur à pression (46) réagissant sur une pression au-dessus de la dite pression de gaz;
- un interrupteur à pression (47) réagissant sur une pression au-dessous de la dite pression de gaz; et
- une conduite (48) pour la connexion avec le vide de l'enveloppe de l'article à nettoyer.

3. Machine selon la revendication 1 ou 2, caractérisée en ce que les moyens (B) comprennent les éléments suivants:
- un réducteur de la pression du gaz (54) pour la fourniture d'une pression gazeuse d'essai;
- une soupape d'essai (76) pour l'ouverture et la fermeture commandées en vue de l'exécution du contrôle de passage libre;
- une vanne d'arrêt actionnée par voie pneumatique (56) pour bloquer l'arrivée du liquide de rinçage dans les canaux à contrôler du dit article;
- des soupapes à commande (58) dans des conduites (50) menant vers les canaux de l'article à nettoyer; et
- un détecteur de débit (60).

4. Machine selon la revendication 3, caractérisée en ce que les dites soupapes à commande (58) sont des soupapes a écrasement de tuyau flexible magnétiquement actionnées et autonettoyantes.

5. Machine selon la revendication 3 ou 4, caractérisée en ce que le détecteur de débit (60) est un interrupteur magnétique à corps flottant qui signale un débit absent ou insuffisant comme étant un dérangement.

6. Machine selon l'une des revendications précédentes, caractérisée par des moyens pour contrôler le passage libre d'au moins un liquide de nettoyage à amener.

7. Machine selon la revendication 6, caractérisée en ce que les dits moyens sont des dispositifs de contrôle de passage libre (72) coopérant avec des pompes (68) montées dans la même conduite.

8. Machine selon l'une des revendications précédentes, caractérisée en ce que les moyens (C) comprennent une vanne d'arrêt actionnée par voie pneumatique (56) qui est périodiquement ouverte et fermée au moyen d'une soupape à commande (52) et qui est montée dans la conduite du liquide de nettoyage (23), et une conduite d'amenée d'air comprimé (55) permettant d'injecter de l'air comprimé dans la conduite de liquide de nettoyage quand la vanne d'arrêt (56) est fermée.

9. Machine selon l'une des revendications précédentes, caractérisée en ce que les moyens (D) comprennent les éléments suivants:
- une pièce de raccordement (91) située dans une paroi (90) de la dite cage (18) et présentant sur le côté intérieur de la cage au moins un raccord pour tuyau flexible (92) pour le raccordement de l'article à nettoyer, sur le côté extérieur au moins une tubulure droite (93), et au moins un alésage traversant;
- un bloc d'accouplement (17) disposé en regard de la machine, comprenant au moins un alésage (95) aligné avec la dite tubulure droite (93), équipé de moyens d'étanchéité (97) côté cage et d'éléments de raccordement (98) côté machine; et
- un cylindre pneumatique à double action (99) pour l'entraînement horizontal du bloc d'accouplement (17) au-dessus des tubulures droites (93) de la pièce de raccordement (91) et en sens inverse afin de libérer celle-ci.

10. Machine selon les revendications 1 et 6, caractérisée en ce qu'un point de mixage (63) est monté dans la conduite d'amenée d'agent désinfectant à titre de liquide de nettoyage, point où se mélangent l'agent désinfectant et de l'eau ultra-pure, et qu'une conduite transportant ce mélange mène par un filtre stérile (67) dans l'intérieur (15) de la machine.

11. Machine selon l'une des revendications précédentes, caractérisée en ce que la cage (18) est exécutée sous forme de caisse qui retient de l'eau de manière à être remplie au cous de l'opération de rinçage.

12. Machine selon la revendication 11, caractérisée en ce que la caisse (18) comprend des repères, et que la machine (10) comporte des capteurs coopérant avec les repères en vue d'une sélection automatique du programme.

13. Machine selon les revendications 11 et 12, caractérisée en ce que la cage (18) présente un clapet latéral qui met en position ouverte au moins un repère hors d'action avec le capteur.

14. Machine selon la revendication 12, caractérisée en ce que les dits repères sont des repères magnétiques.

15. Utilisation de la machine selon l'une des revendications précédentes pour le contrôle et le nettoyage d'endoscopes, en particulier d'endoscopes gastriques.
